# EUROPEAN PATENT APPLICATION

(11) **EP 4 183 448 A1**
(43) Date of publication of application: **24.05.2023**
(21) Application number: 21802586.4
(22) Date of filing: 09.07.2021
(51) Int. Cl.: A61N 5/10

(54) **RADIOTHERAPY SYSTEM AND SAFETY INTERLOCK CONTROL METHOD THEREFOR**

(30) Priority: 20.07.2020 CN 202010701450; 20.07.2020 CN 202010701469
(71) Applicant: Neuboron Therapy System Ltd., Xiamen, Fujian 361026 (CN)
(72) Inventor: HUANG, Yong-yin, Nanjing, Jiangsu 211112 (CN); CHEN, Wei-lin, Nanjing, Jiangsu 211112 (CN)
(74) Representative: Gunzelmann, Rainer
(86) International application number: PCT/CN2021/105370
(87) International publication number: WO 2022/017193

(57) **Abstract**

The invention provides a radiotherapy system and a method for controlling safety interlock thereof, which may improve safety of the radiotherapy system. The radiotherapy system includes a system control module, a beam control module, a beam generating apparatus and an irradiation chamber. The beam generating apparatus includes a charged particle beam generating apparatus and a neutron beam generating portion interacting with a charged particle beam generated by the charged particle beam generating apparatus to generate a therapeutic neutron beam to irradiate into the first irradiation chamber. The controlling method includes the following operations. The beam control module determines, according to the received operation data of the charged particle beam generating apparatus, whether there is a safety problem, or the system control module determines, according to the received operation data of the radiotherapy system, whether there is a safety problem, and the charged particle beam generating apparatus is controlled by the beam control module or by the system control module through the beam control module, to generate the charged particle beam or not, or interact with the neutron beam generating portion.

## Description

### TECHNICAL FIELD

The invention relates to the technical field of radiotherapy, and in particular to a radiotherapy system and a method for controlling safety interlock thereof.

### BACKGROUND

Existing radiotherapy facilities include proton therapy facilities, carbon ion therapy facilities, boron neutron therapy facilities, etc. Most of them use a radiation monitoring component or a shielding door of an irradiation chamber to construct a safety interlock mechanism, that is, if the shielding door of the irradiation chamber is not closed or a radiation monitoring value exceeds a limit during the treatment, a therapeutic beam generating apparatus will automatically shut down immediately to ensure safety of personnel. Although this design may play a protective role, there are still some serious drawbacks. For example, factors involved in safety interlock are relatively singularized. However, in reality, the state of the beam generating apparatus (such as an accelerator, an accelerator auxiliary device, and a target material) will also affect the treatment process. If these devices or components are abnormal during the treatment, the therapeutic effect will be affected directly or damage to personnel and devices will be caused. At the same time, when an emergency occurs during the treatment, for example, the patient has an abnormality and needs personnel to enter the irradiation chamber in time for treatment, it is necessary for personnel to shut down the beam generating apparatus firstly, and then open the door to enter the irradiation chamber for treatment. Entering the irradiation chamber before the beam generating apparatus is completely shut down will expose personnel treating with the emergency to radiation. In addition, it takes time to shut down the beam generating apparatus, delaying the treatment of the emergency, and shutting down the beam generating apparatus forcibly will also have an adverse impact on service life of these treatment facilities.

### SUMMARY

In view of this, embodiments of the invention provide a radiotherapy system and a method for controlling safety interlock thereof, which may improve safety of the radiotherapy system.

According to a first aspect of the embodiments of the invention, there is provided a radiotherapy system, including: an irradiation chamber; a beam generating apparatus, including a charged particle beam generating apparatus and a neutron beam generating portion interacting with a charged particle beam generated by the charged particle beam generating apparatus to generate a therapeutic neutron beam to irradiate into the irradiation chamber; a beam control module, capable of controlling the charged particle beam generating apparatus to generate the charged particle beam and receiving operation data of the charged particle beam generating apparatus; and a system control module, capable of controlling, by the beam control module, the charged particle beam generating apparatus to generate the charged particle beam and receiving operation data of the radiotherapy system including the operation data of the charged particle beam generating apparatus, here the beam control module determines, according to the received operation data of the charged particle beam generating apparatus, whether there is a safety problem, or the system control module determines, according to the received operation data of the radiotherapy system, whether there is a safety problem.

In an embodiment of the invention, the charged particle beam generating apparatus may include a charged particle beam generating portion and a beam transmission portion including a beam direction switching component, and the charged particle beam generating portion generates the charged particle beam to selectively interact with the neutron beam generating portion through the beam direction switching component; the operation data of the charged particle beam generating apparatus includes operation data of the charged particle beam generating portion or operation data of the beam transmission portion including operation data of the beam direction switching component. Furthermore, the operation data of the beam direction switching component may be status data of the beam direction switching component.

In an embodiment of the invention, the charged particle beam generating portion may include an ion source, an accelerator and an accelerator auxiliary device, operation data of the charged particle beam generating portion may include operation data of the ion source, or operation data of the accelerator, or operation data of the accelerator auxiliary device, or a respective fault signal data of the ion source, the accelerator and the accelerator auxiliary device. Furthermore, the ion source may include a gas supply device, an ionization device and a water cooling device. The operation data of the ion source may be pressure of a supplied gas, current and voltage of the ionization device, particle intensity at an outlet of the ion source, and temperature, flow rate and pressure of cooling water of the water cooling device. The accelerator may include a pre-acceleration device, front and rear vacuum chambers, and a high-energy acceleration device. Each of the pre-acceleration device and the high-energy acceleration device may include acceleration pipes, valves and electromagnets. The operation data of the accelerator may be beam intensity in the acceleration pipe and pressure of an insulating gas therein, current of the electromagnet, vacuum degree of each of the front and rear vacuum chambers. The accelerator auxiliary device may include a water cooling device configured to provide the accelerator with cooling water, an air compression device configured to provide compressed air, a gas supply device configured to provide an insulating gas, and a vacuum pump configured to provide vacuum environment. The operation data of the accelerator auxiliary device may be the air pressure of the air compression device, temperature, flow rate and pressure of cooling water of the water cooling device, and pressure of an insulating gas of the gas supply device.

In an embodiment of the invention, the radiotherapy system may further include a charged particle beam generating chamber accommodating the charged particle beam generating portion, a beam transmission chamber accommodating the beam direction switching component, a shielding door of the charged particle beam generating chamber and a shielding door of the beam transmission chamber, the operation data of the radiotherapy system may further include operation data of the shielding door of the charged particle beam generating chamber or operation data of the shielding door of the beam transmission chamber. Furthermore, the operation data of the shielding door may be state data of opening or closing of the shielding door or signal data of opening thereof.

In an embodiment of the invention, the charged particle beam generating apparatus may include a charged particle beam monitoring component, and the operation data of the charged particle beam generating apparatus may include operation data of the charged particle beam monitoring component. Furthermore, the operation data of the charged particle beam monitoring component may be a monitoring value of the charged particle beam monitoring component.

In an embodiment of invention, the beam generating apparatus may further include a neutron beam monitoring component, and the operation data of the radiotherapy system may further include operation data of the neutron beam monitoring component or operation data of the neutron beam generating portion. Furthermore, the operation data of the neutron beam monitoring component may be a monitoring value of the neutron beam monitoring component; the neutron beam generating portion may include a target material, a beam shaping body and a collimator, and the operation data of the neutron beam generating portion may be data of service life of the target material, or data of temperature of the target material, or the model data of the collimator or signal data of an inconsistent collimator.

In an embodiment of the invention, the radiotherapy system may further include a shielding door of the irradiation chamber and a radiation monitoring component arranged in the irradiation chamber, and the operation data of the radiotherapy system may further include operation data of the shielding door of the irradiation chamber or operation data of the radiation monitoring component. Furthermore, the operation data of the shielding door may be state data of opening or closing of the shielding door or signal data of opening thereof, and the operation data of the radiation monitoring component may be a monitoring value of the radiation monitoring component.

In an embodiment of the invention, the radiotherapy system may further include a patient state monitoring component or an activity monitoring component, and the operation data of the radiotherapy system may further include operation data of the patient state monitoring component or operation data of the activity monitoring component. Furthermore, the operation data of the patient state monitoring component may be a monitoring value of the patient state monitoring component or signal data of the patient's abnormality, and the operation data of the activity monitoring component may be a monitoring value of the activity monitoring component or signal data of an abnormal activity.

In an embodiment of the invention, the radiotherapy system may further include a treatment plan module, and the operation data of the radiotherapy system may further include treatment plan data retrieved by the system control module from the treatment plan module.

In an embodiment of the invention, a signal of state of the irradiation chamber may also be provided, and the operation data of the radiotherapy system may further include signal data of the state of the irradiation chamber.

According to a second aspect of the embodiments of the invention, there is provided a method for controlling safety interlock of the above radiotherapy system. The controlling method includes the following operations. The charged particle beam generating apparatus is prevented by the beam control module or by the system control module through the beam control module from generating the charged particle beam, when the beam control module determines, according to the received operation data of the charged particle beam generating apparatus, that there is a safety problem on incoming irradiation of the irradiation chamber, or when the system control module determines, according to the received operation data of the radiotherapy system, that there is a safety problem on incoming irradiation of the irradiation chamber, before the beam generating apparatus generates a therapeutic neutron beam and starts to irradiate the therapeutic neutron beam into the irradiation chamber; or the charged particle beam generating apparatus is controlled by the beam control module or by the system control module through the beam control module to stop generating the charged particle beam, or the charged particle beam generated by the charged particle beam generating apparatus is controlled by the beam control module or by the system control module through the beam control module to stop interacting with the neutron beam generating portion, when the beam control module determines, according to the received operation data of the charged particle beam generating apparatus, that there is a safety problem on the irradiation of the irradiation chamber, or when the system control module determines, according to the received operation data of the radiotherapy system, that there is a safety problem on the irradiation of the irradiation chamber, in the case where the beam generating apparatus generates a therapeutic neutron beam to irradiate the therapeutic neutron beam into the irradiation chamber.

According to a third aspect of the embodiments of the invention, there is provided a method for controlling safety interlock of the radiotherapy system. The radiotherapy system includes a system control module, a beam control module, a beam generating apparatus and a first irradiation chamber. The beam generating apparatus includes a beam direction switching component, and is configured to generate a beam and selectively emit the beam to the first irradiation chamber through the beam direction switching component. The controlling method includes the following operations. When the beam generating apparatus emits the beam to the first irradiation chamber, it is determined, by the beam control module or the system control module according to the received operation data of the radiotherapy system, that there is a safety problem on the irradiation of the first irradiation chamber; the beam direction switching component is controlled by the beam control module or by the system control module through the beam control module to switch the beam away from the first irradiation chamber.

In an embodiment of the invention, the above method for controlling safety interlock may further include the following operations. before the beam generating apparatus emits the beam to the first irradiation chamber, when it is determined, by the beam control module or the system control module according to the received operation data of the radiotherapy system, that there is no safety problem on the incoming irradiation of the first irradiation chamber, the beam generating apparatus is controlled by the beam control module or by the system control module through the beam control module to emit the beam to the first irradiation chamber.

In an embodiment of the invention, the radiotherapy system may further include a second irradiation chamber, here the above method for controlling safety interlock may further include the following operations before controlling, by the beam control module or by the system control module through the beam control module, the beam direction switching component to switch the beam away from the first irradiation chamber. It is determined, by the beam control module or the system control module according to the received operation data of the radiotherapy system, that there is no safety problem in the second irradiation chamber. Here the controlling, by the beam control module or by the system control module through the beam control module, the beam direction switching component to switch the beam away from the first irradiation chamber, may include the following operations. The beam direction switching component is controlled by the beam control module or by the system control module through the beam control module to switch the beam from the first irradiation chamber to the second irradiation chamber.

In an embodiment of the invention, the radiotherapy system may further include a beam collecting apparatus, here the controlling, by the beam control module or by the system control module through the beam control module, the beam direction switching component to switch the beam away from the first irradiation chamber, may include the following operations. The beam direction switching component is controlled by the beam control module or by the system control module through the beam control module to switch the beam from the first irradiation chamber to the beam collecting apparatus.

In an embodiment of the invention, the radiotherapy system may further include a shielding door of the first irradiation chamber, a radiation monitoring component arranged in the first irradiation chamber, a patient state monitoring component and an activity monitoring component. The operation data of the radiotherapy system may further include operation data of the shielding door of the first irradiation chamber or operation data of the radiation monitoring component or operation data of the patient state monitoring component or operation data of the activity monitoring component. Here the determining, by the beam control module or the system control module according to the received operation data of the radiotherapy system, that there is a safety problem on the irradiation of the first irradiation chamber, may include the following operations. It is determined that there is a safety problem on the irradiation of the first irradiation chamber when the system control module receives state data of the opening of the shielding door of the first irradiation chamber; or it is determined that there is a safety problem on the irradiation of the first irradiation chamber when a first monitoring value of the radiation monitoring component received by the system control module exceeds a first preset range; or it is determined that there is a safety problem on the irradiation of the first irradiation chamber when a second monitoring value of the patient state monitoring component received by the system control module exceeds a second preset range or signal data of the patient's abnormality is received; or it is determined that there is a safety problem on the irradiation of the first irradiation chamber when a third monitoring value of the activity monitoring component received by the system control module exceeds a third preset range or signal data of an abnormal activity is received.

In an embodiment of the invention, the beam generating apparatus may further include a charged particle beam generating portion, a first neutron beam generating portion and a beam monitoring component. The beam direction switching component may selectively transmit the charged particle beam generated by the charged particle beam generating portion to the first neutron beam generating portion to irradiate the neutron beam into the first irradiation chamber. The charged particle beam generating portion may include an ion source, an accelerator and accelerator auxiliary device. The radiotherapy system may further include a charged particle beam generating chamber accommodating the charged particle beam generating portion, a beam transmission chamber accommodating the beam direction switching component, a shielding door of the charged particle beam generating chamber, and a shielding door of the beam transmission chamber. Here the operation data of the radiotherapy system may include operation data of the ion source or operation data of the accelerator or operation data of the accelerator auxiliary device or operation data of the beam monitoring component or operation data of the shielding door of the charged particle beam generating chamber or operation data of the shielding door of the beam transmission chamber or operation data of the first neutron beam generating portion or operation data of the beam direction switching component. Here the determining, by the beam control module or the system control module according to the received operation data of the radiotherapy system, that there is a safety problem on the irradiation of the first irradiation chamber, may include the following operations. It is determined that there is a safety problem on the irradiation of the first irradiation chamber, when the beam control module or the system control module determines an abnormality according to the received operation data of the ion source or the operation data of the ion source exceeds a fourth preset range; or it is determined that there is a safety problem on the irradiation of the first irradiation chamber, when the beam control module or the system control module determines an abnormality according to the received operation data of the accelerator or the operation data of the accelerator exceeds a fifth preset range; or it is determined that there is a safety problem on the irradiation of the first irradiation chamber, when the beam control module or the system control module determines an abnormality according to the received operation data of the accelerator auxiliary device or the operation data of the accelerator auxiliary device exceeds a sixth preset range; or it is determined that there is a safety problem on the irradiation of the first irradiation chamber, when a fourth monitoring value of the beam monitoring component received by the beam control module or the system control module exceeds a seventh preset threshold; or it is determined that there is a safety problem on the irradiation of the first irradiation chamber, when the system control module receives state data and signal data of the opening of the shielding door of the charged particle beam generating chamber or of the openin of the shielding door of the beam transmission chamber; or it is determined that there is a safety problem on the irradiation of the first irradiation chamber, when the system control module determines an abnormality according to the received operation data of the first neutron beam generating portion or the operation data of the first neutron beam generating portion exceeds an eighth preset range; or it is determined there is a safety problem on the irradiation of the first irradiation chamber, when the beam control module or the system control module determines an abnormality according to the received operation data of the beam direction switching component.

In an embodiment of the invention, the radiotherapy system may further include a treatment plan module, and the operation data of the radiotherapy system may further include treatment plan data retrieved by the system control module from the treatment plan module. The determining, by the beam control module or the system control module according to the received operation data of the radiotherapy system, that there is a safety problem on the irradiation of the first irradiation chamber, may include the following operations. It is determined that there is a safety problem on the irradiation of the first irradiation chamber, when the system control module determines, according to the comparison of irradiation data of the patient in the first irradiation chamber with the received treatment plan data, that the treatment plan of the patient in the first irradiation chamber is completed.

According to a fourth aspect of the embodiments of the invention, there is provided a radiotherapy system, including: a first irradiation chamber; a beam generating apparatus, including a beam direction switching component and configured to generate a beam and selectively emit the beam to the first irradiation chamber through the bean direction switching component; a system control module, configured to determine, according to the received operation data of the radiotherapy system, that there is a safety problem on the irradiation of the first irradiation chamber, when the beam generating apparatus emits the beam to the first irradiation chamber; and a beam control module, configured to receive a controlling instruction from the system control module and control the beam generating apparatus to switch the beam away from the first irradiation chamber; or determine, according to the received operation data of the radiotherapy system, that there is a safety problem on the irradiation of the first irradiation chamber, when the beam generating apparatus emits the beam to the first irradiation chamber, and control the beam generating apparatus to switch the beam away from the first irradiation chamber.

In an embodiment of the invention, the beam generating apparatus may further include a charged particle beam generating portion, a first neutron beam generating portion and a beam monitoring component. The beam direction switching component may selectively transmits the charged particle beam generated by the charged particle beam generating portion to the first neutron beam generating portion to irradiate the neutron beam into the first irradiation chamber. The charged particle beam generating portion may include an ion source, an accelerator and accelerator auxiliary device. The radiotherapy system may further include a charged particle beam generating chamber accommodating the charged particle beam generating portion, a beam transmission chamber accommodating a beam direction switching component, a shielding door of the charged particle beam generating chamber, and a shielding door of the beam transmission chamber. Each of the shielding door of the charged particle beam generating chamber, the shielding door of the beam transmission chamber and the first neutron beam generating portion is connected to the system control module and performs data interaction with it to determine whether there is a safety problem on the irradiation of the first irradiation chamber. Each of the ion source, the accelerator, the accelerator auxiliary device, the beam monitoring component and the beam direction switching component is connected to the system control module and the beam control module and performs data interaction with them so that the beam control module or the system control module determines whether there is a safety problem in the irradiation of the first irradiation chamber.

In an embodiment of the invention, the radiotherapy system may further include a shielding door of the first irradiation chamber, a radiation monitoring component arranged in the first irradiation chamber, a patient state monitoring component, an activity monitoring component and a treatment plan module. The treatment plan module is configured to store the patient's treatment plan, and each of the treatment plan module, the shielding door of the first irradiation chamber, the radiation monitoring component, the patient state monitoring component and the activity monitoring component is connected to the system control module and perform data interaction with it so that the system control module determines whether there is a safety problem on the irradiation of the first irradiation chamber.

According to technical solutions provided by the embodiments of the invention, the charged particle beam generating apparatus is the source of generating the therapeutic neutron beam. When an abnormality occurs therein, it will directly induce problems on the beam acting on the patient, thereby directly affecting the therapeutic effect or causing damage to personnel and devices, thus the charged particle beam generating apparatus is extremely important as a safety interlock factor. When it is determined, according to the received operation data of the radiotherapy system, that there is a safety problem on the irradiation of the first irradiation chamber, the beam direction switching component is controlled to switch the beam away from the first irradiation chamber, thus the beam may be quickly switched away from the first irradiation chamber without shutting down the beam generating apparatus, so that the safety problem of the first irradiation chamber may be solved in time, and service life of the beam generating apparatus may be increased while improving safety of the radiotherapy system.

### BRIEF DESCRIPTION OF THE DRAWINGS

In order to explain the technical solutions in the embodiments of the invention more clearly, the drawings used in the descriptions of the embodiments will be briefly introduced below. It is apparent that the drawings in the following descriptions are merely some embodiments of the invention, and other drawings may be obtained by those of ordinary skill in the art according to these drawings without paying any creative effort.
FIG. 1 illustrates a block diagram of a radiotherapy system according to an embodiment of the invention.
FIG. 2 illustrates a schematic structural diagram of a radiotherapy system treating a patient according to an embodiment of the invention.
FIG. 3 illustrates a schematic flowchart of a method for controlling safety interlock of a radiotherapy system according to an embodiment of the invention.
FIG. 4 illustrates a block diagram of a radiotherapy system according to another embodiment of the invention.
FIG. 5 illustrates a schematic layout diagram of a radiotherapy system according to another embodiment of the invention.
FIG. 6 illustrates a schematic flowchart of a method for controlling safety interlock of a radiotherapy system according to another embodiment of the invention.
FIG. 7 illustrates a block diagram of a system for controlling safety interlock of a radiotherapy system according to an embodiment of the invention.

### DETAILED DESCRIPTION

The technical solutions in the embodiments of the invention will be clearly and completely described below in combination with the drawings in the embodiments of the invention. It is apparent that the described embodiments are merely a part of the embodiments of the invention, rather than all of the embodiments. According to the embodiments of the invention, all of other embodiments obtained by those of ordinary skill in the art without paying any creative effort shall fall within the protection scope of the invention.

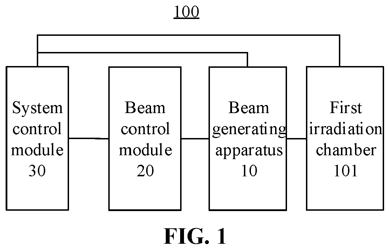

FIG. 1 illustrates a block diagram of a radiotherapy system according to an embodiment of the invention. As illustrated in FIG. 1, the radiotherapy system 100 includes a first irradiation chamber 101, a beam generating apparatus 10, a beam control module 20 and a system control module 30. The beam generating apparatus 10 may generate a therapeutic beam and emit the beam to the first irradiation chamber 101. The first irradiation chamber 101 may perform data interaction with the system control module 30, the beam generating apparatus 10 may perform data interaction with the beam control module 20 or the system control module 30, and the system control module 30 may also perform data interaction with the beam control module 20. The system control module 30 may transmit data input by an operator such as a physician, or received and stored data of the beam generating apparatus 10 and the first irradiation chamber 101, or the like to the beam control module 20, to control the beam generating apparatus 10 to emit a beam to the first irradiation chamber 101.

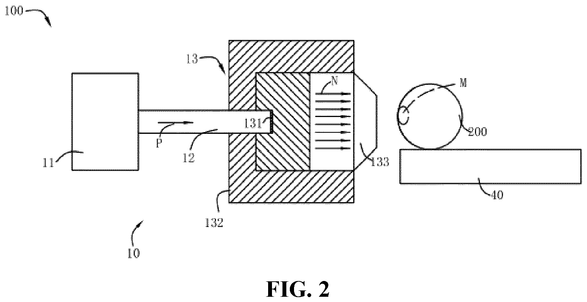

As illustrated in FIG. 2, in an embodiment of the invention, the beam generating apparatus 10 is a neutron beam generating apparatus, and includes a charged particle beam generating portion 11, a beam transmission portion 12 and a first neutron beam generating portion 13. The charged particle beam generating portion 11 may be controlled by the beam control module 20 or by the system control module 30 through the beam control module 20 to generate a charged particle beam P, and the beam transmission portion 12 may be controlled by the beam control module 20 or by the system control module 30 through the beam control module 20 to transmit the charged particle beam P generated by the charged particle beam generating portion 11 to the first neutron beam generating portion 13, and the beam transmission portion 12 is constructed by a transmission pipe. The first neutron beam generating portion 13 corresponds to the first irradiation chamber 101 (not illustrated in the figure), and the charged particle beam P interacts with the first neutron beam generating portion 13 to generate a therapeutic neutron beam N to irradiate to a patient 200 on a treatment table 40 arranged in the first irradiation chamber 101, so as to perform irradiation treatment on the patient 200, such as performing boron neutron capture treatment on tumor cells M in the patient 200. It should be understood that the generated neutron beam may also be used for other purposes, which is not specifically limited in the invention. The beam generating apparatus 10 may also be other radiation generating apparatuses, and the charged particle beam generating portion 11 and the neutron beam generating portion 13 may be replaced or cancelled accordingly. For example, the charged particle beam P generated by the charged particle beam generating portion 11 is directly transmitted to the first irradiation chamber 101 to perform irradiation by the charged particle beam P, and the charged particle beam P is used for therapy or other purposes, which is not limited in the present invention.

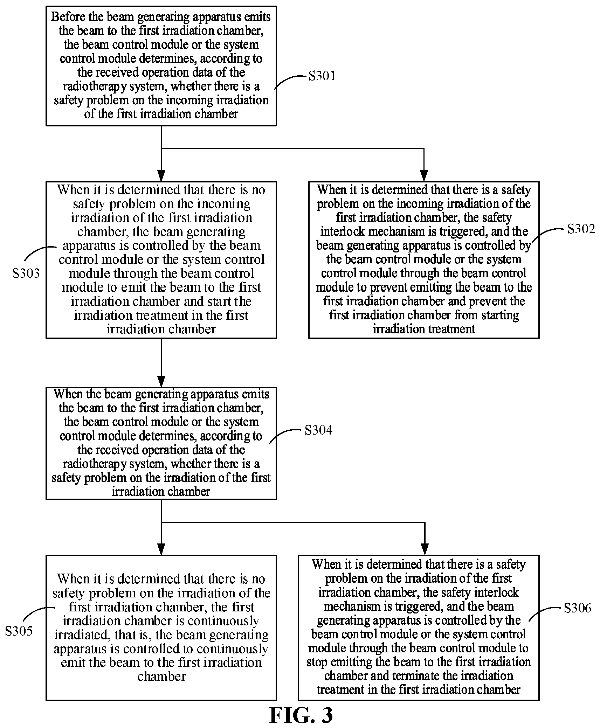

The beam control module 20 or the system control module 30 may receive operation data of the radiotherapy system 100 and determine, according to this operation data, whether there is a safety problem. Specifically, the beam control module 20 may receive operation data of the beam generating apparatus 10 (the charged particle beam generating portion 11 or the beam transmission portion 12), the system control module 30 may receive operation data of the beam generating apparatus 10 or the first irradiation chamber 101. As illustrated in FIG. 3, a method for controlling safety interlock according to an embodiment of the invention is as follows.

In operation S301, the beam control module 20 or the system control module 30 determines, according to the received operation data of the radiotherapy system 100, whether there is a safety problem on incoming irradiation of the first irradiation chamber 101, before the beam generating apparatus 10 emits the beam to the first irradiation chamber 101 (for example, before generating the therapeutic neutron beam N to start to irradiate into the first irradiation chamber 101).

In operation S302, when it is determined, according to the determination result of the operation S301, that there is a safety problem on the incoming irradiation of the first irradiation chamber 101, a safety interlock mechanism is triggered, and the beam generating apparatus 10 may be controlled by the beam control module 20 or by the system control module 30 through the beam control module 20 to prevent emitting the beam to the first irradiation chamber 101 and prevent the first irradiation chamber 101 from starting irradiation treatment, for example, the charged particle beam generating portion 11 is prevented from generating the charged particle beam P.

In operation S303, when it is determined, according to the determination result of the operation S301, that there is no safety problem on the incoming irradiation of the first irradiation chamber 101, the beam generating apparatus 10 may be controlled by the beam control module 20 or by the system control module 30 through the beam control module 20 to emit the beam to the first irradiation chamber 101, start irradiation treatment in the first irradiation chamber 101, for example, the charged particle beam generating portion 11 is controlled to generate the charged particle beam P to interact with the first neutron beam generating portion 13, so as to generate the therapeutic neutron beam N required by the patient 200 currently to be irradiated in the first irradiation chamber 101, to irradiate into the first irradiation chamber 101.

In operation S304, when the beam generating apparatus 10 emits the beam to the first irradiation chamber 101 (for example, when the therapeutic neutron beam N is generated to start to irradiate into the first irradiation chamber 101) according to the operation S303, the beam control module 20 or the system control module 30 determines, according to the received operation data of the radiotherapy system 100, whether there is a safety problem on the irradiation of the first irradiation chamber 101.

In operation S305, when it is determined, according to the determination result of the operation S304, that there is no safety problem on the irradiation of the first irradiation chamber 101, the first irradiation chamber 101 is continuously irradiated, that is, the beam generating apparatus 10 is controlled to continuously emit the beam to the first irradiation chamber 101.

In operation S306, when it is determined, according to the determination result of the operation S304, that there is a safety problem on the irradiation of the first irradiation chamber 101, a safety interlock mechanism is triggered, and the beam generating apparatus 10 may be controlled by the beam control module 20 or by the system control module 30 through the beam control module 20 to stop emitting the beam to the first irradiation chamber 101 and terminate the irradiation treatment in the first irradiation chamber 101, for example, the charged particle beam generating portion 11 is controlled to stop generating the charged particle beam P.

It should be understood that the safety interlock controlling may also be performed only during or before irradiation.

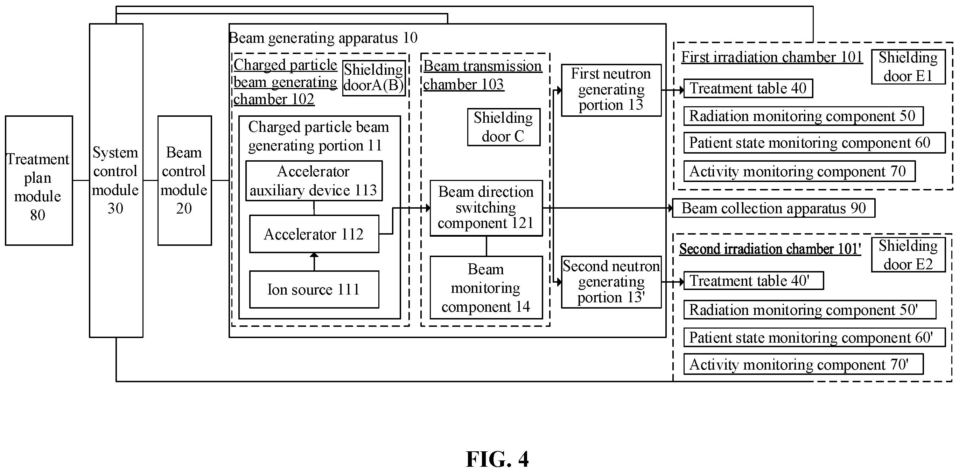

In an embodiment of the invention, as illustrated in FIG. 4, the charged particle beam generating portion 11 includes an ion source 111, an accelerator 112 and an accelerator auxiliary device 113, which is not specifically limited in the invention. The ion source 111 is configured to generate a charged particle, such as H⁻, proton, deuteron, etc. It should be understood that the ion source 111 may be a sputtering ion source, a high-frequency ion source, a dual plasma ion source, a Penning ion source, etc. The invention does not specifically limit types of the ion source. In an embodiment, the ion source 111 includes a gas supply device, an ionization device, a water cooling device, etc., which is not specifically limited in the invention.

The accelerator 112 accelerates the charged particle generated by the ion source 111 to obtain the charged particle beam P with the required energy, such as a proton beam. It should be understood that the accelerator 112 may be a linear accelerator, a cyclotron, a synchrotron, a synchrocyclotron, etc., and the invention does not specifically limit types of the accelerator. In an embodiment, the accelerator 112 includes a pre-acceleration device, front and rear vacuum chambers, a high-energy acceleration device, etc. Each of the pre-acceleration device and the high-energy acceleration device are constructed by acceleration pipes, valves, electromagnets, etc., which is not specifically limited in the invention.

The accelerator auxiliary device 113 may include any auxiliary device configured to provide preconditions for operation of the accelerator 112. In an embodiment, the accelerator auxiliary device 113 includes a water cooling device configured to provide the accelerator with cooling water, an air compression device configured to provide compressed air, a gas supply device configured to provide an insulating gas, and a vacuum pump configured to provide vacuum environment, etc., which is not specifically limited in the invention.

Each of the ion source 111, the accelerator 112 and the accelerator auxiliary device 113 may be connected to the beam control module 20 or the system control module 30 and perform data interaction with them so that the beam control module 20 or the system control module 30 may determine whether there is a safety problem on the irradiation of the first irradiation chamber 101, that is, the operation data of the radiotherapy system 100 includes operation data of the charged particle beam generating portion 11, and the operation data of the charged particle beam generating portion 11 may further include operation data of the ion source 111, operation data of the accelerator 112 and operation data of the accelerator auxiliary device 113. For example, the operation data of the ion source 111, such as pressure of a supplied gas, current and voltage of the ionization device, particle intensity at an outlet of the ion source, and temperature, flow rate and pressure of cooling water of the water cooling device, etc., may be transmitted to the beam control module 20 or the system control module 30; the operation data of the accelerator 112, such as beam intensity in the acceleration pipe and pressure of an insulating gas therein, current of the electromagnet, vacuum degree of each of the front and rear vacuum chambers, etc., may also be transmitted to the beam control module 20 or system control module 30; the operation data of accelerator auxiliary device 113, such as the air pressure of the air compression device, temperature, flow rate and pressure of cooling water of the water cooling device, and pressure of an insulating gas of the gas supply device, etc., may also be transmitted to the beam control module 20 or the system control module 30; a respective fault signal of the ion source 111, the accelerator 112 and the accelerator auxiliary device 113 may also be transmitted to the beam control module 20; and contents of the data interaction are not specifically limited in the invention.

The determining, by the beam control module 20 or the system control module 30 according to the received operation data of the radiotherapy system 100, that there is a safety problem and performing safety interlock, includes the following operations. Before the beam generating apparatus 10 emits the beam to the first irradiation chamber 101 or when the beam generating apparatus 10 emits the beam to the first irradiation chamber 101, in response to the beam control module 20 or the system control module 30 determining an abnormality according to the received operation data of the charged particle beam generating portion 11 or the operation data exceeding a limit, it is determined that there is a safety problem, that is, the safety interlock mechanism is triggered.

Before the beam generating apparatus 10 emits the beam to the first irradiation chamber 101, in response to the beam control module 20 or the system control module 30 determining an abnormality according to the received operation data of the charged particle beam generating portion 11 or the operation data exceeding the limit, which includes: the operation data of the ion source 111, such as the pressure of the supplied gas exceeding a preset range, or the current or voltage of the ionization device exceeding a preset range, or the particle intensity at the outlet of the ion source exceeding a preset range, or the temperature or flow rate or pressure of cooling water of the water cooling device exceeding a preset range, etc.; or the operation data of the accelerator 112, such as the beam intensity in the acceleration pipe or the pressure of the insulating gas therein exceeding a preset range, or the current of the electromagnet exceeding a preset range, or the vacuum degree of each of the front and rear vacuum chambers exceeding a preset range, etc.; or the operation data of the accelerator auxiliary device 113, such as the air pressure of the air compression device exceeding a preset range, or the temperature or flow rate or pressure of cooling water of the water cooling device exceeding a preset range, or the pressure of the insulating gas of the gas supply device exceeding a preset range, then it is determined that there is a safety problem on the incoming irradiation of the first irradiation chamber 101, that is, the safety interlock mechanism is triggered, and the beam generating apparatus 10 may be controlled by the beam control module 20 or by the system control module 30 through the beam control module 20 to prevent emitting the beam to the first irradiation chamber 101 and prevent the first irradiation chamber 101 from starting irradiation treatment. In an embodiment, a respective fault signal of the ion source 111, the accelerator 112 and the accelerator auxiliary device 113 may also be provided, and when the beam control module 20 receives a respective fault signal of the ion source 111, the accelerator 112 and the accelerator auxiliary device 113 to indicate device fault, which is generally a major fault, it is determined that there is a safety problem on the incoming irradiation of the first irradiation chamber 101, that is, the safety interlock mechanism is triggered, the beam control module 20 may prevent the charged particle beam generating portion 11 from generating the charged particle beam P after receiving the fault signal and prevent the first irradiation chamber 101 from starting irradiation treatment.

When the beam generating apparatus 10 emits the beam to the first irradiation chamber 101, in response to the beam control module 20 or the system control module 30 determining an abnormality according to the received operation data of the charged particle beam generating portion 11 or the operation data exceeding the limit, which includes: the operation data of the ion source 111, such as the pressure of the supplied gas exceeding a preset range, or the current or voltage of the ionization device exceeding a preset range, or the particle intensity at the outlet of the ion source exceeding a preset range, or the temperature or flow rate or pressure of cooling water of the water cooling device exceeding a preset range, etc.; or the operation data of the accelerator 112, such as the beam intensity in the acceleration pipe or the pressure of the insulating gas therein exceeding a preset range, or the current of the electromagnet exceeding a preset range, or the vacuum degree of each of the front and rear vacuum chambers exceeding a preset range, etc.; or the operation data of the accelerator auxiliary device 113, such as the air pressure of the air compression device exceeding a preset range, or the temperature or flow rate or pressure of cooling water of the water cooling device exceeding a preset range, or the pressure of the insulating gas of the gas supply device exceeding a preset range, then it is determined that there is a safety problem on the irradiation of the first irradiation chamber 101, the safety interlock mechanism is triggered, and the beam generating apparatus 10 may be controlled by the beam control module 20 or by the system control module 30 through the beam control module 20 to prevent emitting the beam to the first irradiation chamber 101 and terminate the irradiation treatment in the first irradiation chamber 101. When the beam control module 20 receives a respective fault signal of the ion source 111, the accelerator 112 and the accelerator auxiliary device 113, which is generally a major fault, it is determined that there is a safety problem on the irradiation of the first irradiation chamber 101, that is, the safety interlock mechanism is triggered, the beam control module 20 may control the charged particle beam generating portion 11 to stop generating the charged particle beam P after receiving the fault signal, such as cutting off the ion source 111 or shutting down the accelerator 112, to terminate the irradiation treatment in the first irradiation chamber 101.

Since the charged particle beam generating portion is the source of generating the therapeutic neutron beam, and the accelerator is an important device for generating the required charged particle beam, when an abnormality occurs therein, it will directly induce problems on the beam acting on the patient, thereby directly affecting the therapeutic effect or causing damage to personnel and devices, thus the charged particle beam generating portion is extremely important as a safety interlock factor.

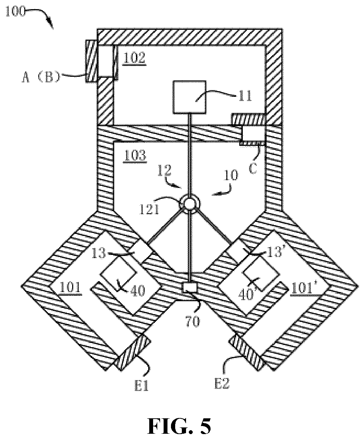

As illustrated in FIG. 5, in another embodiment of the invention, the radiotherapy system 100 may further include a second irradiation chamber 101', and the beam generating apparatus 10 may further include a second neutron beam generating portion 13' corresponding to the second irradiation chamber 101', the beam transmission portion 12 includes a beam direction switching component 121, and the beam transmission portion 12 selectively transmits the charged particle beam P generated by the charged particle beam generating portion 11 to the first neutron beam generating portion 13 or the second neutron beam generating portion 13' through the beam direction switching component 121, to emit a beam into the first irradiation chamber 101 or the second irradiation chamber 101'. It should be understood that the neutron beam N irradiated into the second irradiation chamber 101' may be configured to treat, through irradiation of the neutron beam N, another patient on the treatment bed 40' in the second irradiation chamber 101', and may also be configured for sample detection, etc., which is not be limited in the invention. When the beam generating apparatus 10 is another radiation generating apparatus, the second neutron beam generating portion 13' may also be replaced accordingly, and the beam transmission portion 12 may selectively emit a beam into the first irradiation chamber 101 or the second irradiation chamber 101' through the beam direction switching component 121.

It should be understood that the beam generating apparatus 10 may also have other configurations. For example, when there is a third irradiation chamber, a third neutron beam generating portion may be added to correspond to the third irradiation chamber, and the number of neutron beam generating portions corresponds to the number of irradiation chambers, the number of neutron beam generating portions is not specifically limited in the embodiments of the invention. A charged particle beam generating portion is provided to transmit to each of the neutron beam generating portions, which may effectively reduce system cost. It should be understood that the beam generating apparatus may further include multiple charged particle beam generating portions to transmit to each of the neutron beam generating portions, multiple neutron beams may be simultaneously generated in multiple irradiation chambers for irradiation.

In an embodiment of the invention, the beam direction switching component 121 includes a deflection magnet (not illustrated in the figure) that deflects the direction of the charged particle beam P. When a deflection magnet corresponding to the first irradiation chamber 101 is connected, the beam is guided into the first irradiation chamber 101, which is not specifically limited in the invention. The beam transmission portion 12 may further include a beam adjustment portion (not illustrated in the figure) for the charged particle beam P, and the beam adjustment portion includes a horizontal diverter and a horizontal & vertical diverter configured to adjust axes of the charged particle beam P, a quadrupole electromagnet configured to suppress divergence of the charged particle beam P, and a four-way cutter configured to shape the charged particle beam P, etc. The beam transmission portion 12 may further include a charged particle beam scanning portion (not illustrated in the figure) as required, the charged particle beam scanning portion scans the charged particle beam P and performs irradiation control of the charged particle beam P with respect to the neutron beam generating portions 13, 13', such as controlling an irradiation location of the charged particle beam P with respect to a target material 131 (described below).

The beam transmission portion 12 may be connected to the system control module 30 or the beam control module 20 respectively and perform data interaction with them so that the beam control module 20 or the system control module 30 may determine whether there is a safety problem on the irradiation of the first irradiation chamber 101, that is, the operation data of the radiotherapy system 100 includes operation data of the beam transmission portion 12. For example, vacuum degree of the transmission pipe, a voltage of the magnet, temperature of the magnet, a state (such as a conduction state) data of the beam direction switching component 121, etc. may be transmitted to the system control module 30 or the beam control module 20, and contents of the data interaction are not specifically limited in the invention.

The beam control module 20 or the system control module 30 determines, according to the received operation data of the radiotherapy system 100, that there is a safety problem and performs safety interlock, which includes the following operations Before the beam generating apparatus 10 emits the beam to the first irradiation chamber 101 or when the beam generating apparatus 10 emits the beam to the first irradiation chamber 101, in response to the beam control module 20 or the system control module 30 determining an abnormality according to the received operation data of the beam transmission portion 12 or the operation data exceeding the limit, it is determined that there is a safety problem, that is, the safety interlock mechanism is triggered.

Before the beam generating apparatus 10 emits the beam to the first irradiation chamber 101, in response to the beam control module 20 or the system control module 30 determining an abnormality according to the received operation data of the beam transmission portion 12 or the operation data exceeding the limit, such as the vacuum degree of the transmission pipe exceeding a preset range, or the voltage of the magnet exceeding a preset range, or the temperature of the magnet exceeding a preset range, or the state data of the beam direction switching component 121 indicating that the beam direction switching component 121 does not guide the beam to the first irradiation chamber 101, it is determined that there is a safety problem on the incoming irradiation of the first irradiation chamber 101, that is, a safety interlock mechanism is triggered, the beam generating apparatus 10 may be controlled by the beam control module 20 or by the system control module 30 through the beam control module 20 to prevent emitting the beam to the first irradiation chamber 101 and prevent the first irradiation chamber 101 from starting irradiation treatment.

When the beam generating apparatus 10 emits the beam to the first irradiation chamber 101, in response to the beam control module 20 or the system control module 30 determining an abnormality according to the received operation data of the beam transmission portion 12 or the operation data exceeding the limit, such as the vacuum degree of the transmission pipe exceeding a preset range, or the voltage of the magnet exceeding a preset range, or the temperature of the magnet exceeding a preset range, or the state data of the beam direction switching component 121 indicating that the beam direction switching component 121 does not guide the beam to the first irradiation chamber 101, it is determined that there is a safety problem on the irradiation of the first irradiation chamber 101, that is, a safety interlock mechanism is triggered, the beam generating apparatus 10 may be controlled by the beam control module 20 or by the system control module 30 through the beam control module 20 to stop emitting the beam to the first irradiation chamber 101 and terminate the irradiation treatment in the first irradiation chamber 101.

The beam transmission portion transmits the beam to the irradiation chamber where treatment is required, for example, the beam transmission portion transmits the charged particle beam to the neutron beam generating portion corresponding to the irradiation chamber where treatment is required, so as to generate the therapeutic neutron beam in the irradiation chamber. Occurrence of an abnormality in the beam transmission portion may induce beams to be generated in other irradiation chambers or may be unable to generate the correct beam in the irradiation chamber where treatment is required, resulting in a serious safety accident or affecting the therapeutic effect, so it is also of great significance to take the beam transmission portion as a safety interlock factor.

In an embodiment of the invention, as illustrated in FIG. 2, the first neutron beam generating portion 13 may include a target material 131, a beam shaping body 132 and a collimator 133, which is not specifically limited in the invention. For example, the charged particle beam P generated by the accelerator 112 is irradiated to the target material 131 through the beam transmission portion 11 and interacts with the target material 131 to generate neutrons. The generated neutrons pass through the beam shaping body 132 and the collimator 133 in sequence to form a neutron beam N to irradiate to the patient 200 on the treatment table 40 arranged in the first irradiation chamber 101. The target material 131 may be a metal target material, such as a lithium target material or a beryllium target material, which reacts with a proton line by ⁹Be(p,n)⁹ B or ⁷Li (p, n)⁷ Be nuclear reaction to generate neutrons, the material of the target material 131 is not specifically limited in the invention. There may be multiple collimators 133 with different sizes, shapes, etc., so as to adapt to different to-be-irradiated patients. In an embodiment, an identification mechanism is provided on the collimator 133, and the system control module 30 may automatically identify and obtain model data of the collimator 133, or an operator such as a physician manually inputs the model data of the collimator 133 according to the identification mechanism and transmits it to the system control module 30, or an operator such as a physician determines inconsistency according to the identification mechanism and transmits a signal of an inconsistent collimator to the system control module 30. The specific structures of the target material 131, the beam shaping body 132 and the collimator 133 are not described in detail here. The second neutron beam generating portion 13' may have the same structure as the first neutron beam generating portion 13, which is not specifically limited in the invention.

The first neutron beam generating portion 13 may be connected to the system control module 30 and perform data interaction with it so that the system control module 30 may determine whether there is a safety problem on the irradiation of the first irradiation chamber 101, that is, the operation data of the radiotherapy system 100 includes operation data of the first neutron beam generating portion 13. For example, data such as service life of the target material 131, temperature of the target material 131, the model data of the collimator 133 or signal data of an inconsistent collimator, etc., may be transmitted to the system control module 30, and contents of the data interaction are not specifically limited in the invention.

The beam control module 20 or the system control module 30 determines, according to the received operation data of the radiotherapy system 100, that there is a safety problem and performs safety interlock, which includes the following operations. Before the beam generating apparatus 10 emits the beam to the first irradiation chamber 101 or when the beam generating apparatus 10 emits the beam to the first irradiation chamber 101, in response to the system control module 30 determining an abnormality according to the received operation data of the first neutron beam generating portion 13 or the operation data exceeding a limit, it is determined that there is a safety problem, that is, the safety interlock mechanism is triggered.

Before the beam generating apparatus 10 emits the beam to the first irradiation chamber 101, in response to the system control module 30 determining an abnormality according to the received operation data of the first neutron beam generating portion 13 or the operation data exceeding the limit, for example, the service life of the target material 131 is not enough to complete the next treatment, or the temperature of the target material 131 exceeds a preset range, or the model data of the collimator 133 indicates signal data on an inconsistent patient currently to be irradiated or an inconsistent collimator, it is determined that there is a safety problem on the incoming irradiation of the first irradiation chamber 101, that is, the safety interlock mechanism is triggered. The beam generating apparatus 10 may be controlled by the system control module 30 through the beam control module 20 to prevent emitting the beam to the first irradiation chamber 101 and prevent the first irradiation chamber 101 from starting irradiation treatment.

When the beam generating apparatus 10 emits the beam to the first irradiation chamber 101, in response to the system control module 30 determining an abnormality according to the received operation data of the first neutron beam generating portion 13 or the operation data exceeding the limit, for example, the service life of the target material 131 exceeds a preset range, or the temperature of the target material 131 exceeds a preset range, it is determined that there is a safety problem on the irradiation of the first irradiation chamber 101, that is, the safety interlock mechanism is triggered. The beam generating apparatus 10 may be controlled by the system control module 30 through the beam control module 20 to stop emitting the beam to the first irradiation chamber 101 and terminate the irradiation treatment in the first irradiation chamber 101.

The neutron beam generating portion is critical for generating a therapeutic neutron beam and obtaining beam quality that meets the requirement of treatment, thus the therapeutic effect may be ensured by incorporating the neutron beam generating portion as a safety interlock factor.

In another embodiment of the invention, as illustrated in FIGS. 4 and 5, the radiotherapy system 100 may further include a charged particle beam generating chamber 102 accommodating the charged particle beam generating portion 11, a beam transmission chamber 103 at least partially accommodating the beam transmission portion 12 (for example, accommodating the beam direction switching component 121), a shielding door A(B) of the charged particle beam generating chamber 102 and a shielding door C of the beam transmission chamber 103, which is not specifically limited in the invention. State data of opening or closing of the shielding door may be transmitted to the system control module 30, or the operator may transmit a signal of opening of the shielding door to the system control module 30 according to his/her observed situation. Each of the shielding door A(B) of the charged particle beam generating chamber 102 and the shielding door C of the beam transmission chamber 103 is connected to the system control module 30 and perform data interaction with it so that the system control module 30 may determine whether there is a safety problem on the irradiation of the first irradiation chamber 101, that is, the operation data of the radiotherapy system 100 includes operation data of the shielding door A(B) of the charged particle beam generating chamber 102 and operation data of the shielding door C of the beam transmission chamber 103. For example, the state data of opening or closing of the shielding door A(B) of the charged particle beam generating chamber 102 or the signal data of opening thereof, or the state data of opening or closing of the shielding door C of the beam transmission chamber 103 or the signal data of opening thereof, etc., may be transmitted to the system control module 30, and contents of the data interaction are not specifically limited in the invention. The charged particle beam generating chamber 102 is usually arranged in a two-story space, and the shielding door A of the charged particle beam generating chamber and the shielding door B of the charged particle beam generating chamber are arranged on two floors respectively. It should be understood that other arrangement are also possible.

The beam control module 20 or the system control module 30 determines, according to the received operation data of the radiotherapy system 100, that there is a safety problem on the irradiation of the first irradiation chamber 101 and performs safety interlock, which includes the following operations.

Before the beam generating apparatus 10 emits the beam to the first irradiation chamber 101, in response to the system control module 30 determining an abnormality according to the received operation data of the shielding door A(B) of the charged particle beam generating chamber 102 or the received operation data of the shielding door C of the beam transmission chamber 103, such as the state data of opening of the shielding door A, the shielding door B or the shielding door C, or the signal data of opening of the shielding door A, the shielding door B or the shielding door C, it is determined that there is a safety problem on the incoming irradiation of the first irradiation chamber 101, that is, the safety interlock mechanism is triggered. The beam generating apparatus 10 may be controlled by the system control module 30 through the beam control module 20 to prevent emitting the beam to the first irradiation chamber 101 and prevent the first irradiation chamber 101 from starting irradiation treatment.

When the beam generating apparatus 10 emits the beam to the first irradiation chamber 101, in response to the system control module 30 determining an abnormality according to the received operation data of the shielding door A(B) of the charged particle beam generating chamber 102 or the received operation data of the shielding door C of the beam transmission chamber 103, such as the state data of opening of the shielding door A, the shielding door B or the shielding door C, or the signal data of opening of the shielding door A, the shielding door B or the shielding door C, it is determined that there is a safety problem on the irradiation of the first irradiation chamber 101, that is, the safety interlock mechanism is triggered. The beam generating apparatus 10 may be controlled by the system control module 30 through the beam control module 20 to stop emitting the beam to the first irradiation chamber 101 and terminate the irradiation treatment in the first irradiation chamber 101.

High-energy radiation may be generated when the beam generating apparatus operates. The shielding doors of the charged particle beam generating chamber and the beam transmission chamber are closed during the irradiation treatment to ensure safety of personnel and avoid radiation pollution, thus it is necessary to incorporate the shielding doors as safety interlock factors.

In an embodiment, the beam generating apparatus 10 may further include a beam monitoring component 14. The beam monitoring component 14 may include a charged particle beam monitoring component or a neutron beam monitoring component, which is not specifically limited in the invention. As illustrated in FIG. 4, in the embodiment, the beam monitoring component 14 is a charged particle beam monitoring component and arranged in the beam transmission chamber 103, for example, arranged on an inner wall of the transmission pipe of the beam transmission portion 12, beam intensity is monitored by measuring current of the charged particle beam P, etc. It should be understood that the charged particle beam intensity monitoring component 14 may also be arranged in the charged particle beam generating chamber 102, such as a corresponding device of the ion source 111 or the accelerator 112; voltage, energy of the charged particle beam P, etc., may also be monitored. The neutron beam monitoring component may be arranged in the first neutron beam generating portion 13 to monitor intensity of the neutron beam by measuring radiation generated at the target material 131, and may also be arranged at a neutron beam outlet or in the beam shaping body. The number and arrangement of the beam monitoring components 14 are not specifically limited in the invention.

The beam monitoring component 14 may be connected to the beam control module 20 or the system control module 30 and perform data interaction with them so that the beam control module 20 or the system control module 30 may determine whether there is a safety problem on the irradiation of the first irradiation chamber 101, that is, the operation data of the radiotherapy system 100 includes operation data of the beam monitoring component 14, and the operation data of the beam monitoring component 14 may further include operation data of the charged particle beam monitoring component and operation data of the neutron beam monitoring component. For example, the operation data of the charged particle beam monitoring component, such as the current of the charged particle beam P, etc., may be transmitted to the beam control module 20 or the system control module 30, or the operation data of the neutron beam monitoring component, such as the intensity of the neutron beam or other radiation detection data of the neutron generating portion, etc., may be transmitted to the system control module 30, and contents of the data interaction are not specifically limited in the invention.

The beam control module 20 or the system control module 30 determines, according to the received operation data of the radiotherapy system 100, that there is a safety problem and performs safety interlock, which includes the following operations. Before the beam generating apparatus 10 emits the beam to the first irradiation chamber 101 or when the beam generating apparatus 10 emits the beam to the first irradiation chamber 101, in response to the beam control module 20 or the system control module 30 determining an abnormality according to the received operation data of the beam monitoring component 14 or the operation data exceeding a limit, it is determined that there is a safety problem, that is, the safety interlock mechanism is triggered.

Before the beam generating apparatus 10 emits the beam to the first irradiation chamber 101, in response to the beam control module 20 or the system control module 30 determining an abnormality according to the received operation data of the beam monitoring component 14 or the operation data exceeding the limit, which includes: the operation data of the charged particle beam monitoring component, such as the current of the charged particle beam P exceeding a preset range, it is determined that there is a safety problem on the incoming irradiation of the first irradiation chamber 101, that is, the safety interlock mechanism is triggered. The beam generating apparatus 10 may be controlled by the beam control module 20 or by the system control module 30 through the beam control module 20 to prevent emitting the beam to the first irradiation chamber 101 and prevent the first irradiation chamber 101 from starting irradiation treatment. Or, in response to the system control module 30 determining an abnormality according to the received operation data of the beam monitoring component 14 or the operation data exceeding a limit, which includes: the operation data of the neutron beam monitoring component, such as the intensity of the neutron beam exceeding a preset range, it is determined that there is a safety problem on the incoming irradiation of the first irradiation chamber 101, that is, the safety interlock mechanism is triggered. The beam generating apparatus 10 may be controlled by the system control module 30 through the beam control module 20 to prevent emitting the beam to the first irradiation chamber 101 and prevent the first irradiation chamber 101 from starting irradiation treatment.

When the beam generating apparatus 10 emits the beam to the first irradiation chamber 101, in response to the beam control module 20 or the system control module 30 determining an abnormality according to the received operation data of the beam monitoring component 14 or the operation data exceeding a limit, which includes: the monitoring value of the neutron beam monitoring component, such as the current of the charged particle beam exceeding a preset range, it is determined that there is a safety problem on the irradiation of the first irradiation chamber 101, that is, the safety interlock mechanism is triggered. The beam generating apparatus 10 may be controlled by the beam control module 20 or by the system control module 30 through the beam control module 20 to stop emitting the beam to the first irradiation chamber 101 and terminate the irradiation treatment in the first irradiation chamber 101. Or, in response to the system control module 30 determining an abnormality according to the received operation data of the beam monitoring component 14 or the operation data exceeding a limit, which includes: the monitoring value of the neutron beam monitoring component, such as the intensity of the neutron beam exceeding a preset range, it is determined that there is a safety problem on the irradiation of the first irradiation chamber 101, that is, the safety interlock mechanism is triggered. The beam generating apparatus 10 may be controlled by the system control module 30 through the beam control module 20 to stop emitting the beam to the first irradiation chamber 101 and terminate the irradiation treatment in the first irradiation chamber 101.

It may be directly determined, by the monitoring value of the beam monitoring component, whether the beam meets the requirement or whether the device operates normally, thus it is necessary to incorporate the monitoring value of the beam monitoring component as a safety interlock factor.

In another embodiment of the invention, the radiotherapy system 100 may further include a shielding door E1 of the first irradiation chamber 101, a radiation monitoring component 50 arranged in the first irradiation chamber 101, and the radiation monitoring component 50 is configured to monitor doses of various radiations (such as neutrons and gamma rays) in the first irradiation chamber 101. In an embodiment, boron concentration and tumor dose are calculated by detecting prompt gamma rays emitted by the irradiated site after being irradiated by the neutron beam N. It should be understood that there may be one or more shielding doors E1 of the first irradiation chamber 101, such as including a main shielding door and a secondary shielding door; there may be one or more radiation monitoring components 50 in the first irradiation chamber 101, and the number of the shielding doors E1 and the radiation monitoring components 50 are not specifically limited in the invention. The radiotherapy system 100 may further include a patient state monitoring component 60 and an activity monitoring component 70. The patient state monitoring component 60 may monitor whether the patient's position is shifted, whether the patient's body is unwell, how much boron drug the patient intakes, etc. It should be understood that a patient may trigger a patient abnormality signal on the patient state monitoring component 60 according to the patient's own state, or the operator may trigger a patient abnormality signal on the patient state monitoring component 60 according to the observed situation, or transmit the patient abnormality signal to the system control module 30. The activity monitoring component 70 may monitor whether there are personnel left in the radiation control region such as the irradiation chamber or an abnormal activity of the object through image recognition, a thermal sensor, an infrared sensor, a ultrasonic sensor, a pressure sensor or a laser sensor, etc., and reliability and safety may be ensured by more than two or different types of sensing components. Or, the operator may trigger an activity abnormality signal on the activity monitoring component 70 according to the observed situation, or transmit the activity abnormality signal to the system control module 30. As illustrated in FIG. 4, in the embodiment, the patient state monitoring component 60 and the activity monitoring component 70 are arranged in the first irradiation chamber 101, which is not limited in the invention. It should be understood that the second irradiation chamber may have the same arrangement as the first irradiation chamber.

Each of the shielding door E1 of the first irradiation chamber 101, the radiation monitoring component 50, the patient state monitoring component 60 and the activity monitoring component 70 may be connected to the system control module 30 and perform data interaction with it so that the system control module 30 may determine whether there is a safety problem on the irradiation of the first irradiation chamber 101, that is, the operation data of the radiotherapy system 100 includes operation data of the shielding door E1 of the first irradiation chamber 101, operation data of the radiation monitoring component 50, operation data of the patient state monitoring component 60, and operation data of the activity monitoring component 70. For example, data such as the state data of opening or closing of the shielding door E1 of the first irradiation chamber 101 or the signal data of opening thereof, the monitoring value of the radiation monitoring component 50, the monitoring value or the patient abnormality signal of the patient state monitoring component 60, and the monitoring value or the activity abnormality signal of the activity monitoring component 70, etc., are transmitted to the system control module 30, and contents of the data interaction are not specifically limited in the invention.

The beam control module 20 or the system control module 30 determines, according to the received operation data of the radiotherapy system 100, that there is a safety problem and performs safety interlock, which includes the following operations. Before the beam generating apparatus 10 emits the beam to the first irradiation chamber 101 or when the beam generating apparatus 10 emits the beam to the first irradiation chamber 101, in response to the system control module 30 determining an abnormality or the operation data exceeding a limit according to the received operation data of the shielding door E1 of the first irradiation chamber 101, the operation data of the radiation monitoring component 50, the operation data of the patient state monitoring component 60 or the operation data of the activity monitoring component 70, it is determined that there is a safety problem, that is, the safety interlock mechanism is triggered.

Before the beam generating apparatus 10 emits the beam to the first irradiation chamber 101, in response to the system control module 30 determining an abnormality or the operation data exceeding the limit according to the received operation data of the shielding door E1 of the first irradiation chamber 101, the operation data of the radiation monitoring component 50, the operation data of the patient state monitoring component 60 or the operation data of the activity monitoring component 70, such as the state data of opening of the shielding door E1 of the first irradiation chamber 101 or the signal data of opening thereof, or the monitoring value of the radiation monitoring component 50 exceeding a preset range, or the monitoring value of the patient state monitoring component 60 exceeding a preset range, or the patient abnormality signal of the patient state monitoring component 60, or the monitoring value of the activity monitoring component 70 exceeding a preset range, or the activity abnormality signal of the activity monitoring component 70, it is determined that there is a safety problem on the incoming irradiation of the first irradiation chamber 101, that is, the safety interlock mechanism is triggered. The beam generating apparatus 10 may be controlled by the system control module 30 through the beam control module 20 to prevent emitting the beam to the first irradiation chamber 101 and prevent the first irradiation chamber 101 from starting irradiation treatment.

When the beam generating apparatus 10 emits the beam to the first irradiation chamber 101, in response to the system control module 30 determining an abnormality or the operation data exceeding the limit according to the received operation data of the shielding door E1 of the first irradiation chamber 101, the operation data of the radiation monitoring component 50, the operation data of the patient state monitoring component 60 or the operation data of the activity monitoring component 70, such as the state data of opening of the shielding door E1 of the first irradiation chamber 101 or the signal data of opening thereof, or the monitoring value of the radiation monitoring component 50 exceeding a preset range, or the monitoring value of the patient state monitoring component 60 exceeding a preset range, or the patient abnormality signal data of the patient state monitoring component 60, or the monitoring value of the activity monitoring component 70 exceeding a preset range, or the activity abnormality signal data of the activity monitoring component 70, it is determined that there is a safety problem on the irradiation of the first irradiation chamber 101, that is, the safety interlock mechanism is triggered. The beam generating apparatus 10 may be controlled by the system control module 30 through the beam control module 20 to stop emitting the beam to the first irradiation chamber 101 and terminate the irradiation treatment in the first irradiation chamber 101.

The shielding door of the irradiation chamber is closed during the irradiation treatment to ensure safety of personnel and avoid radiation pollution. The monitoring value of the radiation monitoring component arranged in the irradiation chamber may determine whether the beam meets the requirement or calculate the radiation dose received by the patient. It may be ensured by the patient state monitoring component that the patient is in good state during the treatment or there is no large displacement to ensure the therapeutic effect. It may be ensured by the activity monitoring component that personnel are not exposed to radiation accidentally or there is no abnormal activities of objects to ensure safety of personnel and the device normality, thus it is necessary to incorporate them as safety interlock factors.

In another embodiment of the invention, the radiotherapy system 100 may further include a treatment plan module 80, and the treatment plan module 80 is configured to store the patient's treatment plan. The treatment plan module 80 may be connected to the system control module 30 and perform data interaction with it so that the system control module 30 may determine whether there is a safety problem on the irradiation of the first irradiation chamber 101, that is, the operation data of the radiotherapy system 100 includes treatment plan data retrieved by the system control module 30 from the treatment plan module 80, and contents of the data interaction are not limited in the invention.

The beam control module 20 or the system control module 30 determines, according to the received operation data of the radiotherapy system 100, that there is a safety problem and performs safety interlock, which includes the following operations. Before the beam generating apparatus 10 emits the beam to the first irradiation chamber 101 or when the beam generating apparatus 10 emits the beam to the first irradiation chamber 101, in response to the system control module 30 determining an abnormality according to the received treatment plan data or the treatment plan is completed, it is determined that there is a safety problem, that is, the safety interlock mechanism is triggered.

Before the beam generating apparatus 10 emits the beam to the first irradiation chamber 101, in response to the system control module 30 failing to obtain a compliant treatment plan (consistent with the patient currently to be treated) from the treatment plan module 80, for example, in response to the system control module 30 automatically determining, according to the received treatment plan data, that the treatment plan is wrong, it is determined that there is a safety problem on the incoming irradiation of the first irradiation chamber 101, that is, the safety interlock mechanism is triggered. The beam generating device 10 may be controlled by the system control module 30 through the beam control module 20 to prevent emit the beam to the first irradiation chamber 101 and prevent the first irradiation chamber 101 from starting irradiation treatment. It should be understood that an operator such as a physician may make an artificial judgment (for example, whether the serial number, the patient's information, etc. are consistent) and transmit a signal based on manually confirmed inconsistency to the system control module 30, and the system control module 30 determines, according to the received signal data, that there is a safety problem on the incoming irradiation of the first irradiation chamber 101, that is, the safety interlock mechanism is triggered.

When the beam generating apparatus 10 emits the beam to the first irradiation chamber 101, in response to the system control module 30 determining, according to comparison of the patient's irradiation data in the first irradiation chamber 101 with the received treatment plan data, that the patient's treatment plan in the first irradiation chamber 101 is completed (for example, the treatment duration or the treatment dose in the received treatment plan is reached), it is determined that there is a safety problem on the irradiation of the first irradiation chamber 101, that is, the safety interlock mechanism is triggered. The beam generating apparatus 10 may be controlled by the system control module 30 through the beam control module 20 to stop emitting the beam to the first irradiation chamber 101 and terminate the irradiation treatment in the first irradiation chamber 101.

The dose and duration of radiation received by the patient during the treatment are determined by the treatment plan data. When the treatment plan is wrong, the therapeutic effect will be affected directly or the patient will be endangered, thus effective operation of the radiation treatment may be further ensured by incorporating the treatment plan data as a safety interlock factor.

In another embodiment of the invention, a signal of state of the irradiation chamber (for example, including in-irradiation, to-be-irradiated, in-preparation, unused, etc.) may also be provided, and the signal may be manually confirmed by an operator such as a physician according to the situation of the irradiation chamber, or the signal may also be automatically determined and given by the system control module 30 according to the received data. That is, the operation data of the radiotherapy system may further include the signal data of state of the irradiation chamber. The beam control module 20 or the system control module 30 determines, according to the received operation data of the radiotherapy system 100, that there is a safety problem and performs safety interlock, which includes the following operations. Before the beam generating apparatus 10 emits the beam to the first irradiation chamber 101, in response to the system control module 30 determining that the first irradiation chamber 101 is not in a to-be-irradiated state, according to the received signal data of state of the first irradiation chamber 101, for example, the state of the irradiation chamber 101 indicates an in-preparation signal or a unused signal, it is determined that there is a safety problem on the incoming irradiation of the first irradiation chamber 101, that is, the safety interlock mechanism is triggered. The beam generating apparatus 10 may be controlled by the system control module 30 through the beam control module 20 to prevent emitting the beam to the first irradiation chamber 101 and prevent the first irradiation chamber 101 from starting irradiation treatment.

In another embodiment of the invention, the radiotherapy system may further include a beam collection apparatus 90. The beam collection apparatus 90 may be a container buried in a wall to collect the beam when the beam is not required, and the beam direction switching component 121 transmits the charged particle beam P generated by the charged particle beam generating portion 11 to the beam collection apparatus 90. The beam generating apparatus 10 may be controlled by the beam control module 20 or by the system control module 30 through the beam control module 20 to stop emitting the beam to the first irradiation chamber 101, which may be in a manner that the charged particle beam generating portion 11 is controlled to stop generating the charged particle beam P, or that the charged particle beam P generated by the charged particle beam generating portion 11 is controlled to stop interacting with the first neutron beam generating portion 13, that is, the beam generating apparatus 10 is controlled to allow the beam direction switching component 121 to switch the beam away from the first irradiation chamber 101, for example, the charged particle beam P generated by the charged particle beam generating portion 11 is controlled to interact with the second neutron beam generating portion 13' through the beam direction switching component 121 so as to generate the neutron beam N irradiated into the second irradiation chamber 101', thereby switching the beam from the first irradiation chamber 101 to the second irradiation chamber 101'; or, the charged particle beam P generated by the charged particle beam generating unit 11 is controlled not to interact with the first and second neutron beam generating portions 13, 13' through the beam direction switching component 121, and the charged particle beam P is directly transmitted to the beam collection apparatus 90, thereby switching the beam from the first irradiation chamber 101 to the beam collection apparatus 90. The selection of the above manner may be determined automatically by the beam control module 20 or the system control module 30 according to the received operation data of the radiotherapy system 100, or may be input manually by the operator prompted after the safety interlock mechanism is triggered. In an embodiment, when the factor triggering the safety interlock is not related to the beam generating apparatus 10, such as opening of the shielding door of the irradiation chamber or the patient's abnormality, the beam may be selected to switch away from the first irradiation chamber 101; alternatively, when the factor triggering the safety interlock is related to the beam generating apparatus 10, such as an accelerator fault, the charged particle beam generating portion 11 may be controlled to stop generating the charged particle beam P. It should be understood that there may also be other settings, which is not limited in the invention.

In an embodiment, before the beam is switched from the first irradiation chamber 101 to the second irradiation chamber 101', the method for controlling safety interlock may further include the following operations. The beam control module 20 or the system control module 30 determines, according to the received operation data of the radiotherapy system 100, whether there is a safety problem on the second irradiation chamber 101' (whether the second irradiation chamber 101' is in an unused state and whether the shielding door of the second irradiation chamber 101' is closed). When it is determined that the second irradiation chamber 101' is in an unused state and the shielding door of the second irradiation chamber 101' is closed, the beam is switched from the first irradiation chamber 101 to the second irradiation chamber 101'; when it is determined that the second irradiation chamber 101 is not in a unused state and the shielding door of the second irradiation chamber 101' is not closed, the operation of switching the beam from the first irradiation chamber 101 to the second irradiation chamber 101' is not performed and a prompt is given. Specifically, a part of the beam direction switching component 121 corresponding to the first irradiation chamber 101 may be disconnected, and a part of the beam direction switching component 121 corresponding to the second irradiation chamber 101' may be connected, so as to realize that the beam is switched from the first irradiation chamber 101 to the second irradiation chamber 101'.

According to the technical solutions provided by the embodiments of the invention, when the beam control module 20 or the system control module 30 determines, according to the received operation data of the radiotherapy system 100, that there is a safety problem on the irradiation of the first irradiation chamber 101, the beam generating apparatus 10 is controlled by the beam control module 20 or the system control module 30 to switch the beam away from the first irradiation chamber 101, so that the beam may be quickly switched away from the first irradiation chamber 101 without shutting down the beam generating apparatus, so that the safety problem of the first irradiation chamber 101 may be solved in time, and service life of the beam generating apparatus 10 may be increased while improving safety of the radiotherapy system 100. In addition, the first irradiation chamber 101 and the second irradiation chamber 101' share a beam generating apparatus 10, which improves the utilization rate of the beam generating apparatus 10 and meets the requirement of multiple irradiation chambers cooperating simultaneously for safety interlock protection. All of the above optional technical solutions may be combined in any way to form optional embodiments of the invention, which is not repeated here.

In an embodiment of the invention, the principle of the safety interlock mechanism is briefly described by example of the shielding door and the beam direction switching component 121 cooperating with each other to form a safety interlock factor. The shielding door includes the shielding door of the charged particle beam generating chamber 102 (such as the shielding door A and the shielding door B), the shielding door C of the beam transmission chamber 103, and the shielding door of the irradiation chamber (the shielding door E1 of the first irradiation chamber and the shielding door E2 of the second irradiation chamber). The beam direction switching component 121 includes a deflection magnet D1 and a deflection magnet D2, which are configured to guide the irradiation beam into the first irradiation chamber 101 and the second irradiation chamber 101', respectively. The connection of the deflection magnet D1 and the connection of the deflection magnet D2 are mutually exclusive, for example, when the deflection magnet D1 is connected, the deflection magnet D2 is disconnected, and when the deflection magnet D2 is connected, the deflection magnet D1 is disconnected.

The start of the radiation treatment in the irradiation chamber: each of the shielding door A and the shielding door B of the charged particle beam generating chamber 102, and the shielding door C of the beam transmission chamber 103 must be closed; in addition, at least one of the deflection magnets must be connected and a corresponding shielding door of the irradiation chamber is closed, for example, the deflection magnet D1 is connected and the shielding door E1 of the first irradiation chamber is closed; or the deflection magnet D2 is connected and the shielding door E2 of the second irradiation chamber is closed.

In short, when each of the shielding door A, the shielding door B and the shielding door C is closed, the deflection magnet D1 is connected and the corresponding shielding door E1 of the first irradiation chamber 101 is closed, a beam generating condition is reached for the charged particle beam generating portion 11; or when each of the shielding door A, the shielding door B and the shielding door C is closed, the deflection magnet D2 is connected and the corresponding shielding door E2 of the second irradiation chamber 101' is closed, a beam generating condition is reached for the charged particle beam generating portion 11. That is, after the system control module receives an instruction of starting the irradiation of the first or second irradiation chamber and performs the above safety interlock judgment to determine that there is no safety problem on the irradiation of the irradiation chamber, the system control module controls the charged particle beam generating portion 11 to generate the charged particle beam P (the ion source 111, the accelerator 112, and the accelerator auxiliary device 113 operate to reach the beam generating state), the charged particle beam P interacts with the corresponding neutron beam generating portion to generate a neutron beam to irradiate into the irradiation chamber, then the irradiation treatment in the irradiation chamber is started.

The termination of the irradiation treatment in the irradiation chamber: during execution of the irradiation treatment in the first irradiation chamber 101, when at least one shielding door (for example, at least one of the shielding doors A, B, C or E1) is opened or the state of the deflection magnet D1 is abnormal (for example, it is accidentally switched from the connection state to the disconnection state), it is determined that there is a safety problem on the irradiation of the first irradiation chamber 101. As long as the deflection magnet D1 is disconnected or the charged particle beam generating portion 11 stops generating the charged particle beam P (such as shutting down the accelerator 112 or cutting off the ion source 111), the beam is switched away from the first irradiation chamber 101, and the irradiation treatment in the first irradiation chamber 101 is terminated. When the deflection magnet D1 is disconnected to switch the beam away from the first irradiation chamber 101, it is determined that the second irradiation chamber 101' is in an unoccupied state and the shielding door is closed, then the deflection magnet D2 may be connected, and the beam is switched from the first irradiation chamber 101 to the second irradiation chamber 101'; both the deflection magnets D1 and D2 may also be disconnected, and the beam is switched from the first irradiation chamber to the beam collection apparatus 90.

During execution of the irradiation treatment in the second irradiation chamber 101', when at least one shielding door (for example, at least one of the shielding doors A, B, C or E2) is opened or the state of the deflection magnet D1 is abnormal (for example, it is accidentally switched from the connection state to the disconnection state), it is determined that there is a safety problem on the second irradiation chamber 101'. As long as the deflection magnet D2 is disconnected or the charged particle beam generating portion 11 stops generating the charged particle beam P (such as shutting down the accelerator 112 or cutting off the ion source 111), the beam is switched away from the second irradiation chamber 101', and the irradiation treatment in the second irradiation chamber 101' is terminated. When the deflection magnet D2 is disconnected to switch the beam away from the second irradiation chamber 101', it is determined that the first irradiation chamber 101 is in an unoccupied state and the shielding door is closed, then the deflection magnet D 1 may be connected, and the beam is switched from the second irradiation chamber 101' to the first irradiation chamber 101; both the deflection magnets D1 and D2 may also be disconnected, and the beam is switched from the second irradiation chamber 101' to the beam collection apparatus 90.

In order to explain the principle of the safety interlock mechanism simply and clearly, the above descriptions only take the shielding door and the deflection magnet used as the safety interlock factors as an example. It should be understood that in addition to the shielding door and the deflection magnet, other factors (for example, ion source, accelerator, accelerator auxiliary device, neutron beam generating portion, beam monitoring component, radiation monitoring component, treatment plan module, etc.) mentioned here may also be added to cooperate together to form a safety interlock mechanism, which is not limited in the invention. By incorporating a variety of devices and components as the safety interlock factors, safety of the radiotherapy system is effectively improved and effective usage of the radiotherapy system is strengthened.

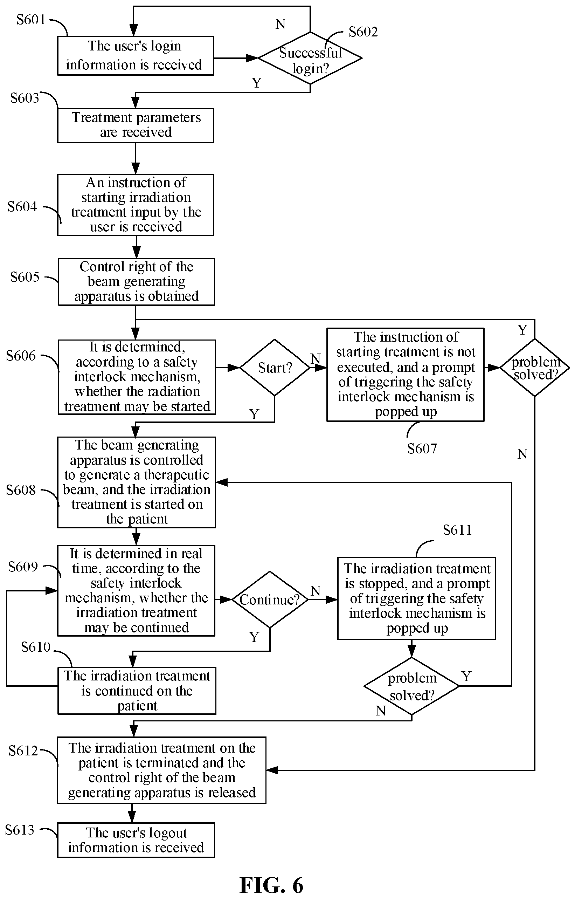

FIG. 6 illustrates a schematic flowchart of a method for controlling safety interlock of a radiotherapy system according to another embodiment of the invention, which takes the neutron irradiation treatment in the first irradiation chamber as an example. The method for controlling safety interlock may be executed by a system 700 for controlling safety interlock in the radiotherapy system. The system 700 for controlling safety interlock may include control software and a carrier for executing a control program, and may also include a user input interface and a feedback display interface, and may further include device connection ports of a processor module, a data acquisition module, a beam generating apparatus or an irradiation chamber, etc., which is not specifically limited in the embodiments of the invention. As illustrated in FIG. 6, the method for controlling safety interlock includes the following operations.

In operation S601, the user's login information is received.

In operation S602, after receiving the user's login information in the operation S601, it is determined whether the user has successfully logged in.

When the user has not successfully logged in, the process returns to the operation S601, and when the user has successfully logged in, operation S603 is performed.

In operation S603, treatment parameters are received.

Before and after receiving the treatment parameters, the user may also verify the treatment device or the patient's state. The treatment parameters may be manually input by the user, or may be treatment parameters in the treatment plan data retrieved from the treatment plan module, which is not limited in the invention.

In operation S604, after receiving the treatment parameters in the operation S603, an instruction input by the user to start the irradiation treatment in the first irradiation chamber 101 is received.

In operation S605, after receiving the instruction input by the user to start the irradiation treatment in the first irradiation chamber 101 in the operation S604, control right of the beam generating apparatus 10 is obtained.

In operation S606, after the operation S605, it is determined, according to a safety interlock mechanism, whether the radiation treatment may be started on the first irradiation chamber 101.

Specifically, it is determined whether there is a safety problem on the incoming irradiation of the first irradiation chamber 101. When there is a safety problem on the incoming irradiation of the first irradiation room 101, operation S607 is executed; when there is no safety problem on the incoming irradiation of the first irradiation chamber 101 and treatment may be started, operation S608 is executed.

In operation S607, the instruction of starting treatment is not executed, and a prompt of triggering the safety interlock mechanism is popped up.

For example, the prompt may be operation data exceeding a limit, device fault, deflection magnet being not connected, shielding door being not closed, insufficient service life of the target material, inconsistent collimator, patient's abnormality, wrong treatment plan, etc., which is not limited in the invention. The user solves the safety problem according to the prompt. When the problem is solved, for example, the corresponding shielding door is closed, etc., the user manually selects determination of solving the problem, then the process returns to the operation S606, and performs the safety interlock judgment before starting the irradiation again; when the problem cannot be solved temporarily, such as a serious device fault, the user manually selects determination of not solving the problem, and then operation S612 is executed to terminate the radiation treatment on the patient and release the control right of the beam generating apparatus 10.

In operation S608, the beam generating apparatus 10 is controlled to generate a therapeutic beam, and the irradiation treatment is started on the patient 200 in the first irradiation chamber 101.

Specifically, the charged particle beam generating portion 11 is controlled to generate a charged particle beam P, and the beam transmission portion 12 is controlled to transmit the charged particle beam P generated by the charged particle beam generating portion 11 to the first neutron beam generating portion 13, and the charged particle beam P interacts with the first neutron beam generating portion 13 to generate a therapeutic neutron beam N to irradiate the patient 200 on the treatment table 40 arranged in the first irradiation chamber 101, so as to perform the irradiation treatment on the patient 200.

In operation S609, after starting the irradiation treatment on the patient in the first irradiation chamber 101 in the operation S608, it is determined in real time, according to the safety interlock mechanism, whether the irradiation treatment may be continued on the first irradiation chamber 101.

That is, it is determined in real time whether there is a safety problem on the irradiation of the first irradiation chamber 101. When there is no safety problem on the irradiation of the first irradiation chamber 101, operation S610 is executed; and when there is a safety problem on the irradiation of the first irradiation chamber 101, operation S611 is executed.

In operation S610, the irradiation treatment is continued on the patient 200 in the first irradiation chamber 101, and the process returns to the operation S609 to continue the judgment.

In operation S611, the irradiation treatment on the patient 200 in the first irradiation chamber 101 is stopped, and a prompt of triggering the safety interlock mechanism is popped up.

For example, the prompt may be operation data exceeding a limit, device fault, deflection magnet being not connected, shielding door being opened, patient's abnormality, completion of the treatment plan, etc., which is not limited in the invention. The user solves the safety problem according to the prompt. When the problem is solved, for example, the corresponding shielding door is closed, etc., the user manually selects determination of solving the problem, then the process returns to the operation S608, and starts the irradiation treatment on the patient 200 in the first irradiation chamber 101 again; when the problem cannot be solved temporarily, such as a serious device fault, the user manually selects determination of not solving the problem, and then operation S612 is executed to terminate the radiation treatment on the patient 200 in the first irradiation chamber 101 and release the control right of the beam generating apparatus 10.

In operation S612, the irradiation treatment on the patient 200 in the first irradiation chamber 101 is terminated and the control right of the beam generating apparatus 10 is released.

In operation S613, after the irradiation treatment is terminated in the operation S612, the user's logout information is received.

It should be understood that in the above operations, after the safety interlock determines that there is a safety problem, a prompt of triggering the safety interlock mechanism may also be popped up firstly according to factors triggering the safety interlock, and the user may determine, according to the prompt, whether the irradiation treatment may be started or continued. When some operation data exceeds a respective preset range by a small margin, physicians, etc., determine according to experience that the operation data are still within a safe range and the irradiation treatment may be started or continued.

According to the technical solutions provided by the embodiments of the invention, the safety interlock mechanism formed according to multiple safety interlock factors is configured to monitor whether there is a safety problem before and during the radiation treatment, thereby improving safety of the radiotherapy system.

All of the above optional technical solutions may be combined in any way to form an optional embodiment of the invention, which is not repeated here.

For the implementation of the function and effect of each module in the above apparatus, please refer to the implementation of corresponding steps in the above method for details, which is not repeated here.

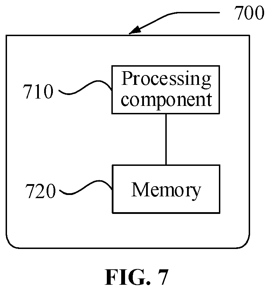

FIG. 7 illustrates a block diagram of a system 700 for controlling safety interlock of a radiotherapy system according to an embodiment of the invention.

Referring to FIG. 7, the system 700 for controlling safety interlock includes a processing component 710 which further includes one or more processors, and a memory resource represented by a memory 720 which is configured to store instructions that may be executed by the processing component 710, such as application programs. The application programs stored in the memory 720 may include one or more modules each of which corresponding to a set of instructions. In addition, the processing component 710 is configured to execute instructions to execute the above method for controlling safety interlock of the radiotherapy system.

The system 700 for controlling safety interlock may further include a power supply component which is configured to perform power management of the system 700 for controlling safety interlock, a wired or wireless network interface which is configured to connect the system 700 for controlling safety interlock to a network, and an input and output (I/O) interface. The system 700 for controlling safety interlock may operate according to an operation system stored in the memory 720, such as Windows Server^{™}, Mac OS X^{™}, Unix^{™}, Linux^{™}, FreeBSD^{™} or the like.

In a non-transitory computer-readable storage medium, when instructions in a storage medium are executed by the processor of the system 700 for controlling safety interlock, the system 700 for controlling safety interlock may execute any one of the above methods for controlling safety interlock of the radiotherapy system.

Those of ordinary skill in the art may be aware that the units and algorithm steps of the examples described in combination with the embodiments disclosed here may be implemented by electronic hardware or a combination of computer software and electronic hardware. Whether these functions are executed by hardware or software, depends on specific applications and design constraint conditions of the technical solutions. Professional technicians may use different methods for each specific application to implement the described functions, but such implementation should not be considered as going beyond the scope of the invention.

Those skilled in the art may clearly understand that for the convenience and conciseness of description, specific working processes of the above system, apparatus and unit may refer to corresponding processes in the above method embodiments, which is not repeated here.

In several embodiments provided in the invention, it should be understood that the disclosed system, apparatus and method may be implemented in other ways. For example, the above apparatus embodiments are only illustrative. For example, the division of the units is based on a logical function division only, and there may be other divisions in actual implementation, for example, multiple units or components may be combined or integrated into another system, or some features may be ignored or may not be implemented. In addition, the displayed or discussed mutual coupling or direct coupling or communication connection may be indirect coupling or communication connection through some interfaces, apparatuses or units, and may be in electrical, mechanical or other forms.

The units described as separate components may be or may not be physically separated, and the components displayed as units may be or may not be physical units, that is, they may be located in one place, or they may be distributed on multiple network units. Some or all of the units may be selected according to actual needs to achieve the objectives of solutions in the embodiment.

In addition, the functional units in the embodiments of the invention may be integrated into one processing unit, or each of the units may exist alone physically, or two or more units may be integrated into one unit.

When the function is implemented in the form of a software functional unit and sold or used as an independent product, it may be stored in a computer-readable storage medium. Based on such an understanding, the technical solutions of the invention substantially or parts making contributions to the conventional art or a part of the technical solution may be embodied in form of software product, and the computer software product is stored in a storage medium, including multiple instructions configured to enable a piece of computer equipment (which may be a personal computer, a server, network equipment or the like) to execute all or part of steps of the method in each embodiment of the invention. The abovementioned storage medium includes: various media capable of storing program check codes such as a U disk, a mobile hard disk, a Read-Only Memory (ROM), a Random Access Memory (RAM), a magnetic disk or an optical disk, etc.

In addition, it should be noted that the combination of technical features in the invention is not limited to the combination described in the claims of the invention or the combination described in the specific embodiments. All the technical features described in the invention may be freely combined or combined in any way, unless there is a contradiction between them.

It should be noted that the above-listed embodiments are only specific embodiments of the invention. It is apparent that the invention is not limited to the above embodiments, and there are many similar changes. When those skilled in the art directly derive or conceive of all the modifications from the disclosure of the invention, they shall belong to the protection scope of the invention.

It should be understood that qualifiers such as first, second, etc., mentioned in the embodiments of the invention are only intended to describe the technical solutions in the embodiments of the invention more clearly, and may not be intended to limit the protection scope of the invention.

The above are only preferred embodiments of the invention and not intended to limit the protection scope of the invention. Any modification, equivalent replacement, improvement, etc., made within the spirit and principle of the invention should be included in the protection scope of the invention.

## Claims

1. A radiotherapy system, comprising:
a first irradiation chamber;
a beam generating apparatus, comprising a charged particle beam generating apparatus and a first neutron beam generating portion interacting with a charged particle beam generated by the charged particle beam generating apparatus to generate a therapeutic neutron beam to irradiate into the first irradiation chamber;
a beam control module, capable of controlling the charged particle beam generating apparatus to generate the charged particle beam and receiving operation data of the charged particle beam generating apparatus; and
a system control module, capable of controlling, by the beam control module, the charged particle beam generating apparatus to generate the charged particle beam and receiving operation data of the radiotherapy system comprising the operation data of the charged particle beam generating apparatus,
wherein the beam control module determines, according to the received operation data of the charged particle beam generating apparatus, whether there is a safety problem, or the system control module determines, according to the received operation data of the radiotherapy system, whether there is a safety problem.

2. The radiotherapy system of claim 1, wherein the charged particle beam generating apparatus comprises a charged particle beam generating portion and a beam transmission portion comprising a beam direction switching component, and the charged particle beam generating portion generates the charged particle beam to selectively interact with the first neutron beam generating portion through the beam direction switching component, to generate the therapeutic neutron beam to irradiate into the first irradiation chamber.

3. The radiotherapy system of claim 2, wherein when the beam generating apparatus irradiates the neutron beam into the first irradiation chamber and the beam control module or the system control module determines that there is a safety problem on irradiation of the first irradiation chamber, the beam control module or the system control module is capable of controlling, through the beam control module, the beam direction switching component to switch the neutron beam away from the first irradiation chamber.

4. The radiotherapy system of claim 2, wherein the operation data of the charged particle beam generating apparatus comprises operation data of the charged particle beam generating portion or operation data of the beam transmission portion comprising operation data of the beam direction switching component.

5. The radiotherapy system of claim 2, wherein the charged particle beam generating portion comprises an ion source, an accelerator and an accelerator auxiliary device, operation data of the charged particle beam generating portion comprises operation data of the ion source, or operation data of the accelerator, or operation data of the accelerator auxiliary device, or a respective fault signal data of the ion source, the accelerator and the accelerator auxiliary device.

6. The radiotherapy system of claim 2, further comprising a charged particle beam generating chamber accommodating the charged particle beam generating portion, a beam transmission chamber accommodating the beam direction switching component, a shielding door of the charged particle beam generating chamber and a shielding door of the beam transmission chamber, the operation data of the radiotherapy system further comprises operation data of the shielding door of the charged particle beam generating chamber or operation data of the shielding door of the beam transmission chamber.

7. The radiotherapy system of claim 1, wherein the charged particle beam generating apparatus comprises a charged particle beam monitoring component, and the operation data of the charged particle beam generating apparatus comprises operation data of the charged particle beam monitoring component.

8. The radiotherapy system of claim 1, wherein the beam generating apparatus further comprises a neutron beam monitoring component, and the operation data of the radiotherapy system further comprises operation data of the neutron beam monitoring component or operation data of the first neutron beam generating portion.

9. The radiotherapy system of claim 1, further comprising a shielding door of the first irradiation chamber and a radiation monitoring component arranged in the first irradiation chamber, and the operation data of the radiotherapy system further comprises operation data of the shielding door of the first irradiation chamber or operation data of the radiation monitoring component.

10. The radiotherapy system of claim 1, further comprising a patient state monitoring component or an activity monitoring component, and the operation data of the radiotherapy system further comprises operation data of the patient state monitoring component or operation data of the activity monitoring component.

11. The radiotherapy system of claim 1, further comprising a treatment plan module, and the operation data of the radiotherapy system further comprises treatment plan data retrieved by the system control module from the treatment plan module.

12. A method for controlling safety interlock of the radiotherapy system of any one of claims 1-11, comprising:
preventing, by the beam control module or by the system control module through the beam control module, the charged particle beam generating apparatus from generating the charged particle beam, when the beam control module determines, according to the received operation data of the charged particle beam generating apparatus, that there is a safety problem on incoming irradiation of the first irradiation chamber, or when the system control module determines, according to the received operation data of the radiotherapy system, that there is a safety problem on incoming irradiation of the first irradiation chamber, before the beam generating apparatus generates a therapeutic neutron beam and starts to irradiate the therapeutic neutron beam into the first irradiation chamber; or
controlling, by the beam control module or by the system control module through the beam control module, the charged particle beam generating apparatus to stop generating the charged particle beam, or controlling, by the beam control module or by the system control module through the beam control module, the charged particle beam generated by the charged particle beam generating apparatus to stop interacting with the first neutron beam generating portion, when the beam control module determines, according to the received operation data of the charged particle beam generating apparatus, that there is a safety problem on the irradiation of the first irradiation chamber, or when the system control module determines, according to the received operation data of the radiotherapy system, that there is a safety problem on the irradiation of the first irradiation chamber, in the case where the beam generating apparatus generates a therapeutic neutron beam to irradiate the therapeutic neutron beam into the first irradiation chamber.

13. The method for controlling safety interlock of claim 12, wherein the charged particle beam generating apparatus comprises a charged particle beam generating portion and a beam transmission portion comprising a beam direction switching component, and the charged particle beam generating portion generates the charged particle beam to selectively interact with the first neutron beam generating portion through the beam direction switching component, to generate the therapeutic neutron beam to irradiate into the first irradiation chamber, and the controlling, by the beam control module or by the system control module through the beam control module, the charged particle beam generated by the charged particle beam generating apparatus to stop interacting with the first neutron beam generating portion, comprises controlling, by the beam control module or by the system control module through the beam control module, the beam direction switching component to switch the neutron beam away from the first irradiation chamber.

14. The method for controlling safety interlock of claim 13, wherein the radiotherapy system further comprises a second irradiation chamber, and the method further comprises before controlling, by the beam control module or by the system control module through the beam control module, the beam direction switching component to switch the neutron beam away from the first irradiation chamber:
determining, by the beam control module or the system control module according to the received operation data of the radiotherapy system, that there is no safety problem in the second irradiation chamber,
wherein the controlling, by the beam control module or by the system control module through the beam control module, the beam direction switching component to switch the neutron beam away from the first irradiation chamber, comprises:
controlling, by the beam control module or by the system control module through the beam control module, the beam direction switching component to switch the neutron beam from the first irradiation chamber to the second irradiation chamber.

15. The method for controlling safety interlock of claim 13, wherein the radiotherapy system further comprises a beam collecting apparatus, wherein the controlling, by the beam control module or by the system control module through the beam control module, the beam direction switching component to switch the neutron beam away from the first irradiation chamber, comprises:
controlling, by the beam control module or by the system control module through the beam control module, the beam direction switching component to switch the neutron beam from the first irradiation chamber to the beam collecting apparatus.
